# EUROPEAN PATENT APPLICATION

(11) **EP 1 739 132 A2**
(43) Date of publication of application: **03.01.2007**
(21) Application number: 06017493.5
(22) Date of filing: 23.08.2006
(51) Int. Cl.: C08L 83/12, C08G 77/46, A61K 8/894

(54) **Cosmetic or pharmaceutical compositions comprising modified polyorganosiloxanes**

(30) Priority: 14.07.2006 US 487271
(71) Applicant: CLARIANT INTERNATIONAL LTD., 4132 Muttenz (CH)
(72) Inventor: Klug, Peter, Dr., 63762 Grossostheim (DE); Haala, Sabine, 63457 Hanau (DE); Müller, Carsten, 60486 Frankfurt am Main (DE); Meder, Markus, Dr., 65779 Kelkheim (DE); Landtiser, Richard, Gainesville, FL 32607 (US)
(74) Representative: Paczkowski, Marcus

(57) **Abstract**

Linear organopolysiloxane-polyoxyalkylene copolymers of the formula (I) 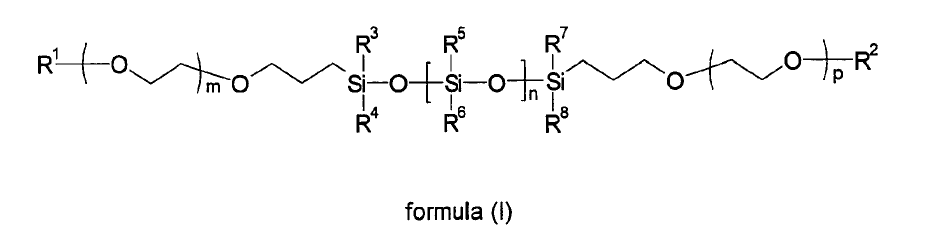 are described, in which
R¹ and R², independently of one another, are a straight-chain or branched, saturated alkyl group having 6 to 22 carbon atoms or are a straight-chain or branched, mono- or polyunsaturated alkenyl group having 6 to 22 carbon atoms,
R³, R⁴, R⁵, R⁶, R⁷ and R⁸, independently of one another, are a straight-chain or branched, saturated alkyl group having 1 to 30 carbon atoms, a straight-chain or branched, mono- or polyunsaturated alkenyl group having 2 to 30 carbon atoms or a phenyl radical,
m and p are numbers from 4 to 50 and
n is a number from 3 to 90.

The linear organopolysiloxane-polyoxyalkylene copolymers of the formula (I) can be used in an advantageous way in cosmetic or pharmaceutical compositions.
Moreover, they are suitable particularly for the cleansing and care of the hair and the skin, as emulsifier in emulsions, preferably in O/W or W/O emulsions, and as conditioners.

## Description

The invention relates to linear organopolysiloxane-polyoxyalkylene copolymers, to cosmetic or pharmaceutical compositions or preparations comprising one or more of these linear organopolysiloxane-polyoxyalkylene copolymers and to the use of these linear organopolysiloxane-polyoxyalkylene copolymers as emulsifier.

The use of emulsifier systems based on polysiloxane is known. US 4,988,504 describes polysiloxane emulsifiers which consist of R₂SiO_{2/2} units, polyoxyalkylene groups as side chains and siloxane chains as end members.

EP 1 400 554 discloses dimethylpolysiloxanes with polyoxyethylene and/or polyoxypropylene side chains which can be used as W/O emulsifier.

EP 407 089 describes polydimethylsiloxane with polyoxyethylene and/or polypropylene side chains and molecular weights of from 10 000 to 20 000 g/mol. One disadvantage of these silicone emulsifiers is that they are of high viscosity, are moderately soluble in solvents and can only be handled in dilution.

EP 819 426 discloses dimethylpolysiloxanes with terminal polyoxyalkylene groups, where the polyoxyalkylene groups may also be terminally capped with short-chain alkyl groups, for example a methyl group or ethyl group. These polysiloxane groups are characterized by low viscosity and can advantageously be used as W/O emulsifier.

The use options of the hitherto known emulsifier systems based on polysiloxane are essentially restricted to W/O emulsions due to their inadequate solubility in organic solvents and organic ingredients, and also their inadequate interface activity toward oils.

It was therefore an object to provide new types of Si emulsifier systems which are compatible with organic oils, are suitable both as water-in-oil and also as oil-in-water emulsifier, exhibit good sensory properties for the skin and for the hair and do not adversely affect the foaming behavior of the formulations.

Surprisingly, this object was achieved by linear organopolysiloxane copolymers comprising dimethylpolysiloxane and polyalkylene groups bonded in the main chain which are terminally capped with long-chain alkyl radicals.

The invention provides linear organopolysiloxane-polyoxyalkylene copolymers of the formula (I) 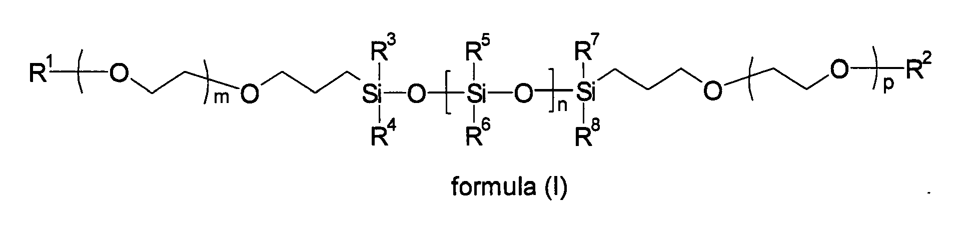 in which
R¹ and R², independently of one another, are a straight-chain or branched, saturated alkyl group having 6 to 22 carbon atoms or are a straight-chain or branched, mono- or polyunsaturated alkenyl group having 6 to 22 carbon atoms,
R³, R⁴, R⁵, R⁶, R⁷ and R⁸, independently of one another, are a straight-chain or branched, saturated alkyl group having 1 to 30 carbon atoms, a straight-chain or branched, mono- or polyunsaturated alkenyl group having 2 to 30 carbon atoms or a phenyl radical,
m and p are numbers from 4 to 50 and
n is a number from 3 to 90.

A preferred subject-matter of the invention are linear organopolysiloxane-polyoxyalkylene copolymers of the formula (I) in which
R¹ and R², independently of one another, are a straight-chain or branched, saturated alkyl group having 12 to 22 carbon atoms, preferably 16 to 22 carbon atoms, or a straight-chain or branched, mono- or polyunsaturated alkenyl group having 12 to 22 carbon atoms, preferably 16 to 22 carbon atoms, and
R³, R⁴, R⁵, R⁶, R⁷ and R⁸ are in each case a methyl group.

A further preferred subject-matter of the invention are linear organopolysiloxane-polyoxyalkylene copolymers of the formula (I) in which
m and p are numbers from 5 to 40, preferably 6 to 30 and particularly preferably 7 to 25 and
n is a number from 10 to 60, preferably 20 to 40 and particularly preferably 22 to 30.

A particularly preferred subject-matter of the invention are linear organopolysiloxane-polyoxyalkylene copolymers according to formula (I) in which
R¹ and R² are cetearyl,
R³, R⁴, R⁵, R⁶, R⁷ and R⁸ are in each case a methyl group,
m and p are numbers from 20 to 30, preferably 25, and
n is a number from 20 to 30, preferably 25.

A further particularly preferred subject-matter of the invention are linear organopolysiloxane-polyoxyalkylene copolymers of the formula (I) in which
R¹ and R² are cetearyl,
R³, R⁴, R⁵, R⁶, R⁷ and R⁸ are in each case a methyl group,
m and p are numbers from 20 to 30, preferably 25, and
n is a number from 70 to 90, preferably 80.

The linear organopolysiloxane-polyoxyalkylene copolymers of the formula (I) according to the invention can be synthesized in accordance with customary methods by catalytic hydrosilylation of allyl ethers from fatty alcohol alkoxylates with linear silanes under reflux in the presence of a Pt catalyst. The allyl ethers from fatty alcohol alkoxylates used as starting materials are accessible by reacting alkoxylated fatty alcohols with allyl halides.

The organopolysiloxane-polyoxyalkylene copolymers of the formula (I) according to the invention are colorless to pale beige solids which are readily soluble in all customary organic solvents and are readily dispersible in water. They can be incorporated easily into formulations and produce an esthetic appearance. They are compatible with organic oils, solvents and with ingredients customary in cosmetic products. In particular, the organopolysiloxane-polyoxyalkylene copolymers of the formula (I) according to the invention are characterized by marked hydrophilic behavior and have an excellent emulsifying effect, both in preparations based on water-in-oil, i.e. W/O; and on oil-in-water, i.e. O/W.

Moreover, they impart very good sensory properties for the skin and for the hair to the cosmetic cleansing and care products. They improve the combability of the hair and exhibit excellent substantivity, and also good conditioning and moisturizing effect and are thus valuable constituents of haircare and hair-cleansing compositions, hair colorants, skincare and skin-cleansing compositions, sunscreen compositions, deodorants, antiperspirants and decorative cosmetics.

The present invention therefore further provides the use of one or more linear organopolysiloxane-polyoxyalkylene copolymers of the formula (I) in cosmetic or pharmaceutical compositions or preparations, and also cosmetic or pharmaceutical compositions or preparations comprising one or more linear organopolysiloxane-polyoxyalkylene copolymers of the formula (I).

The compositions according to the invention may, for example, be aqueous-alcoholic, aqueous-surface-active or alcoholic compositions, or compositions based on oil, inclusive compositions based on oil in anhydrous form, or emulsions, suspensions or dispersions.

In a preferred embodiment, the cosmetic or pharmaceutical compositions according to the invention are in aqueous-alcoholic, alcoholic or aqueous-surface-active form or represent compositions based on oil, in particular anhydrous compositions based on oil, or are in the form of an emulsion, suspension or dispersion and, more particularly, in the form of fluids, foams, sprays, gels, cream gels, mousse, lotions, creams or powders.

In a particularly preferred embodiment, the cosmetic or pharmaceutical compositions according to the invention are in the form of emulsions.

The emulsions may either be water-in-oil emulsions (W/O emulsions) or oil-in-water emulsions (O/W emulsions), microemulsions, nanoemulsions and multiple emulsions, preferably water-in-oil emulsions or oil-in-water emulsions.

The emulsions can be prepared in a known manner, i.e. for example by cold, hot, hot/cold or PIT emulsification.

The cosmetic or pharmaceutical compositions according to the invention in the form of an emulsion comprise the one or more organopolysiloxane-polyoxyalkylene copolymers of the formula (I) preferably in amounts of from 0.01 to 30% by weight, particularly preferably from 0.05 to 10% by weight and especially preferably from 0.1 to 5% by weight, based on the finished compositions.

In a further preferred embodiment, the compositions according to the invention are oil-in-water emulsions with an oil content of from 5 to 95% by weight, preferably 15 to 75% by weight and particularly preferably 25 to 85% by weight.

In a further preferred embodiment, the compositions according to the invention are water-in-oil emulsions with a water content of from 5 to 95% by weight, preferably 15 to 75% by weight and particularly preferably 25 to 65% by weight.

Good substantivity, conditioning effect, and shine- and volume-imparting effects of the above-described organopolysiloxane-polyoxyalkylene copolymers of the formula (I) are utilized according to the invention for producing hair-treatment compositions.

In a further preferred embodiment, the compositions according to the invention are therefore in the form of hair-treatment compositions, preferably shampoos, hair conditioners, hair treatments, styling compositions, hair rinses, volume spray, styling fluid, hair mousse, hair gel, setting compositions, hair spray, mousse, hair oils and end fluids.

Conditioning effects and good skin sensory properties of skincare and skin-cleansing compositions are achieved through the above-described organopolysiloxane-polyoxyalkylene copolymers of the formula (I).

In a further preferred embodiment, the cosmetic or pharmaceutical compositions according to the invention are rinse-off products, in particular make-up removers, shower baths, shower gels or foam baths.

In a further preferred embodiment, the cosmetic or pharmaceutical compositions according to the invention are leave-on products, in particular day creams, night creams, care creams, nutrient creams, body lotions, ointments or lipcare compositions.

Further preferred leave-on products are decorative cosmetics, in particular make-ups, eyeshadows, lipsticks or mascara.

In a further preferred embodiment, the cosmetic and pharmaceutical compositions according to the invention are sunscreen compositions. These comprise one or more UV filters.

In a further preferred embodiment, the cosmetic and pharmaceutical compositions according to the invention are deodorants or antiperspirants, in particular in the form of sprays, sticks, gels, creams or lotions.

In a further preferred embodiment, the cosmetic and pharmaceutical compositions according to the invention are surfactant-free compositions, in particular surfactant-free solid compositions or surfactant-free emulsions.

In a further preferred embodiment, the cosmetic or pharmaceutical compositions according to the invention are additives for permanent waving compositions, in particular conditioners.

The cosmetic or pharmaceutical compositions according to the invention on an aqueous-alcoholic, alcoholic or aqueous-surface-active basis comprise the organopolysiloxane-polyoxyalkylene copolymers of the formula (I) preferably in the amounts of from 0.01 to 30% by weight, particularly preferably from 0.2 to 15% by weight and especially preferably from 0.5 to 10% by weight, based on the finished compositions.

The cosmetic or pharmaceutical compositions according to the invention in anhydrous form based on oils comprise the organopolysiloxane-polyoxyalkylene copolymers of the formula (I) preferably in amounts of from 0.01 to 80% by weight, particularly preferably from 0.05 to 60% by weight and especially preferably from 0.1 to 50% by weight, based on the finished compositions.

For the compositions according to the invention on an aqueous-alcoholic or alcoholic basis, all mono- or polyhydric alcohols are suitable. Preference is given to alcohols having 1 to 4 carbon atoms, such as ethanol, propanol, isopropanol, n-butanol, isobutanol, t-butanol or glycerol, and alkylene glycols, in particular propylene glycol, butylene glycol or hexylene glycol, and mixtures of said alcohols. Further preferred alcohols are polyethylene glycols with a relative molecular mass below 2000. In particular, use of polyethylene glycol with a relative molecular mass between 200 and 600 and of polyethylene glycol with a relative molecular mass between 400 and 600 is preferred.

The oil-based compositions according to the invention can preferably comprise:
hydrocarbon oils with linear or branched, saturated or unsaturated C₇-C₄₀-carbon chains, for example dodecane, isododecane, cholesterol, hydrogenated polyisobutylenes, docosanes, hexadecane, isohexadecane, paraffins and
isoparaffins, but also triglycerides of animal and vegetable origin, for example beef tallow, pig fat, goose grease, perhydrosqualene, lanolin, sunflower oil, maize oil, soya oil, rice oil, jojoba oil, babusscu oil, pumpkin oil, grapeseed oil, sesame oil, walnut oil, apricot oil, macadamia oil, avocado oil, sweet almond oil, lady's smock oil, castor oil, olive oil, peanut oil, rapeseed oil and coconut oil and synthetic oils, such as purcellin oil, linear and/or branched fatty alcohols and fatty acid esters, preferably Guerbet alcohols having 6 to 18, preferably 8 to 10, carbon atoms; esters of linear (C₆-C₁₃)-fatty acids with linear (C₆-C₂₀)-fatty alcohols; esters of branched (C₆-C₁₃)-carboxylic acids with linear (C₆-C₂₀)-fatty alcohols, esters of linear (C₆-C₁₈)-fatty acids with branched alcohols, in particular 2-ethylhexanol; esters of linear and/or branched fatty acids with polyhydric alcohols (such as e.g. dimerdiol or trimerdiol) and/or Guerbet alcohols; alcohol esters of C₁-C₁₀-carboxylic acids or C₂-C₃₀-dicarboxylic acids, esters, such as dioctyl adipate, diisopropyl dimer dilineolate; propylene glycol/dicaprylate or waxes, such as beeswax, paraffin wax or microcrystalline waxes, optionally in combination with hydrophilic waxes, such as, for example, cetylstearyl alcohol; fluorinated and perfluorinated oils;
monoglycerides of C₁-C₃₀-carboxylic acids, diglycerides of C₁-C₃₀-carboxylic acids, triglycerides of C₁-C₃₀-carboxylic acids, for example triglycerides of caprylic/capric acids, ethylene glycol monoesters of C₁-C₃₀-carboxylic acids, ethylene glycol diesters of C₁-C₃₀-carboxylic acids, propylene glycol monoesters of C₁-C₃₀-carboxylic acids, propylene glycol diesters of C₁-C₃₀-carboxylic acids, and propoxylated and
ethoxylated derivatives of the abovementioned classes of compound. The carboxylic acids can comprise linear or branched alkyl groups or aromatic groups. By way of example, mention may be made of diisopropyl sebacate, diisopropyl adipate, isopropyl myristate, isopropyl palmitate, myristyl propionate, ethylene glycol distearate, 2-ethylhexyl palmitate, isodecyl neopentanoate, di-2-ethylhexyl maleate, cetyl palmitate, myristyl myristate, stearyl stearate, cetyl stearate, behenyl behenate, dioctyl maleate, dioctyl sebacate, cetyl octanoate, diisopropyl dilinoleate, caprylic/capryl triglyceride, PEG-6 caprylic/capryl triglyceride, PEG-8 caprylic/capryl triglyceride, cetyl ricinoleate, cholesterol hydroxystearate, cholesterol isostearate, C₁-C₃₀-monoesters and polyesters of glycerol, for example glyceryl tribehenate, glyceryl stearate, glyceryl palmitate, glyceryl distearate, glyceryl dipalmitate, C₁-C₃₀-carboxylic monoesters and polyesters of sugar, for example glucose tetraoleate, glucose tetraesters of soya oil fatty acid, mannose tetraesters of soya oil fatty acid, galactose tetraesters of oleic acid, arabinose tetraesters of linoleic acid, xylose tetralinoleate, galactose pentaoleate, sorbitol tetraoleate, sorbitol hexaesters of unsaturated soya oil fatty acid, xylitol pentaoleate, sucrose tetraoleate, sucrose pentaoleate, sucrose hexaoleate, sucrose heptaoleate, sucrose oleate.

The silicone oils available are preferably dimethylpolysiloxanes and cyclomethicones, polydialkylsiloxanes R₃SiO(R₂SiO)ₓSiR₃, where R is methyl or ethyl, particularly preferably methyl, and x is a number from 2 to 500, for example the dimethicones available under the trade names Vicasil (General Electric Company), Dow Corning 200, Dow Corning 225, Dow Corning 200 (Dow Corning Corporation), trimethylsiloxysilicates [((CH₂)₃SiO)_{1/2}]ₓ[SiO₂]_{y}, where x is a number from 1 to 500 and y is a number from 1 to 500, dimethiconols R₃SiO[R₂SiO]ₓSiR₂OH and HOR₂SiO[R₂SiO]ₓSiR₂OH, where R is methyl or ethyl and x is a number up to 500, polyalkylarylsiloxanes, for example the polymethylphenylsiloxanes available under the trade names SF 1075 Methylphenyl Fluid (General Electric Company) and 556 Cosmetic Grade Phenyl Trimethicone Fluid (Dow Corning Corporation) and SilCare Silicone 15M grades (Clariant), polydiarylsiloxanes, silicone resins, cyclic silicones and amino-, fatty acid-, alcohol-, polyether-, epoxy-, fluorine- and/or alkyl-modified silicone compounds, such as, for example, SilCare Silicone 31 M grades and SilCare Silicone 41M15, and polyether siloxane copolymers.

As further auxiliaries and additives, the cosmetic or pharmaceutical compositions according to the invention can comprise surfactants, further emulsifiers, cationic polymers, thickeners, film formers, antimicrobial active ingredients, astringents, antioxidants, UV light protection filters, pigments/micropigments, gelling agents, and further additives customary in cosmetics, such as, for example, superfatting agents, moisturizing agents, silicones, stabilizers, conditioning agents, glycerol, preservatives, pearlizing agents, dyes, fragrance and perfume oils, solvents, hydrotropes, opacifiers, fatty alcohols, substances with a keratolytic and keratoplastic effect, antidandruff agents, biogenic active ingredients (local anesthetics, antibiotics, antiphlogistics, antiallergics, corticosteroids, sebostatics), vitamins, Bisabolol^{®}, Allantoin^{®}, Phytantriol^{®}, Panthenol^{®}, AHA acids (alpha-hydroxy acids), plant extracts, for example aloe vera and proteins.

Anionic washing-active substances which may be mentioned are preferably:
C₁₀-C₂₀-alkyl and alkylene carboxylates, alkyl ether carboxylates, fatty alcohol sulfates, fatty alcohol ether sulfates, alkylamide sulfates and sulfonates, fatty acid alkylamide polyglycol ether sulfates, alkane sulfates, alkanesulfonates, and hydroxyalkanesulfonates, olefinsulfonates, acyl esters of isethionates, α-sulfo fatty acid esters, alkylbenzenesulfonates, alkylphenol glycol ether sulfonates, sulfosuccinates, sulfosuccinic monoesters and diesters, fatty alcohol ether phosphates, protein-fatty acid condensation products, alkyl monoglyceride sulfates and sulfonates, alkyl glyceride ether sulfonates, fatty acid methyl taurides, fatty acid sarcosinates, sulforicinoleates, amphoacetates or amphoglycinates, acyl glutamates. These compounds and their mixtures are used in the form of their water-soluble or water-dispersible salts, for example the sodium, potassium, magnesium, ammonium, mono-, di- and triethanolammonium and analogous alkylammonium salts.

The content of anionic surfactants in the compositions according to the invention is preferably 1 to 30% by weight, particularly preferably 5 to 25% by weight and especially preferably 10 to 22% by weight, based on the finished compositions.

Suitable cationic surfactants are, for example, quaternary ammonium salts, such as di(C₁₀-C₂₄-alkyl)dimethylammonium chloride or bromide, preferably di(C₁₂-C₁₈-alkyl)dimethylammonium chloride or bromide; C₁₀-C₂₄-alkyldimethylethylammonium chloride or bromide; C₁₀-C₂₄ alkyltrimethylammonium chloride or bromide, preferably cetyltrimethylammonium chloride or bromide and C₂₀-C₂₂-alkyltrimethylammonium chloride or bromide; C₁₀-C₂₄-alkyldimethylbenzylammonium chloride or bromide, preferably C₁₂-C₁₈-alkyldimethylbenzylammonium chloride; N-(C₁₀-C₁₈-alkyl)pyridinium chloride or bromide, preferably N-(C₁₂-C₁₆-alkyl)pyridinium chloride or bromide; N-(C₁₀-C₁₈-alkyl)isoquinolinium chloride, bromide or monoalkyl sulfate; N-(C₁₂-C₁₈-alkyl)polyoylaminoformylmethyl)pyridinium chloride; N-(C₁₂-C₁₈-alkyl)-N-methylmorpholinium chloride, bromide or monoalkyl sulfate; N-(C₁₂-C₁₈-alkyl)-N-ethylmorpholinium chloride, bromide or monoalkyl sulfate; C₁₆-C₁₈-alkylpentaoxyethylammonium chloride; diisobutylphenoxyethoxyethyldimethylbenzylammonium chloride; salts of N,N-diethylaminoethylstearylamide and -oleylamide with hydrochloric acid, acetic acid, lactic acid, citric acid, phosphoric acid; N-acylaminoethyl-N,N-diethyl-N-methylammonium chloride, bromide or monoalkyl sulfate and N-acylaminoethyl-N,N-diethyl-N-benzylammonium chloride, bromide or monoalkyl sulfate, where acyl is preferably stearyl or oleyl.

The content of cationic surfactants in the compositions according to the invention is preferably 0.1 to 10% by weight, particularly preferably 0.2 to 7% by weight and especially preferably 0.5 to 5% by weight, based on the finished compositions.

Suitable nonionic surfactants which can be used as washing-active substances are preferably fatty alcohol ethoxylates (alkylpolyethylene glycols); alkylphenol polyethylene glycols; alkyl mercaptan polyethylene glycols; fatty amine ethoxylates (alkylaminopolyethylene glycols); fatty acid ethoxylates (acyl polyethylene glycols); polypropylene glycol ethoxylates (Pluronics^{®}); fatty acid amide polyethylene glycols; N-alkyl-, N-alkoxypolyhydroxy fatty acid amide, in particular fatty acid N-methyl-glucamides, sucrose esters; polyglycol ethers, alkyl polyglycosides, phosphoric esters (mono-, di- and triphosphoric esters ethoxylated and nonethoxylated).

The content of nonionic surfactants in the compositions according to the invention (e.g. in the case of rinse-off products) is preferably 1 to 20% by weight, particularly preferably 2 to 10% by weight and especially preferably 3 to 7% by weight, based on the finished compositions.

Preferred amphoteric surfactants are: N-(C₁₂-C₁₈-alkyl)-β-aminopropionates and N-(C₁₂-C₁₈-alkyl)-β-iminodipropionates as alkali metal and mono-, di- and trialkylammonium salts; N-acylaminoalkyl-N,N-dimethylacetobetaine, preferably N-(C₈-C₁₈-acyl)aminopropyl-N,N-dimethylacetobetaine; C₁₂-C₁₈-alkyldimethylsulfopropylbetaine; amphoteric surfactants based on imidazoline (trade name: Miranol^{®}, Steinapon^{®}), preferably the sodium salt of 1-(β-carboxymethyloxyethyl)-1-(carboxymethyl)-2-laurylimidazolinium; amine oxides, e.g. C₁₂-C₁₈-alkyldimethylamine oxide, fatty acid amidoalkyldimethylamine oxide.
The content of amphoteric surfactants in the compositions according to the invention is preferably 0.5 to 20% by weight and particularly preferably 1 to 10% by weight, based on the finished compositions.

Furthermore, foam-boosting cosurfactants from the group consisting of alkylbetaines, alkylamidobetaines, aminopropionates, aminoglycinates, imidazoliniumbetaines and sulfobetaines, amine oxides and fatty acid alkanolamides or polyhydroxyamides can be used in the compositions according to the invention.

Preferred surfactants in the compositions according to the invention are alkyl ether sulfates, alkyl sulfates, in particular lauryl sulfate, alkylbetaines, in particular cocoamidopropylbetaine, amphoacetates, acyl glutamates, in particular sodium cocoyl glutamate, alkyl ether sulfosuccinates, in particular disodium laureth sulfosuccinate and coconut fatty acid diethanolamide.

The total amount of surfactants used in the compositions according to the invention is preferably 1 to 70% by weight, particularly preferably 10 to 40% by weight and especially preferably 12 to 35% by weight, based on the finished compositions.

Compositions according to the invention in the form of emulsions can be produced without further emulsifier or else comprise one or more emulsifiers. These emulsifiers can be chosen from the group of nonionic, anionic, cationic or amphoteric emulsifiers.

Suitable nonionogenic coemulsifiers are preferably addition products of from 0 to 30 mol of ethylene oxide and/or 0 to 5 mol of propylene oxide onto linear fatty alcohols having 8 to 22 carbon atoms, onto fatty acids having 12 to 22 carbon atoms, onto alkylphenols having 8 to 15 carbon atoms in the alkyl group and onto sorbitan or sorbitol esters; (C₁₂-C₁₈) fatty acid monoesters and diesters of addition products of from 0 to 30 mol of ethylene oxide onto glycerol; glycerol monoesters and diesters and sorbitan monoesters and diesters of saturated and unsaturated fatty acids having 6 to 22 carbon atoms and optionally ethylene oxide addition products thereof; addition products of from 15 to 60 mol of ethylene oxide onto castor oil and/or hydrogenated castor oil; polyol and, in particular, polyglycerol, esters, such as, for example, polyglycerol polyricinoleate and polyglycerol poly-12-hydroxystearate. Likewise preferably suitable are ethoxylated fatty amines, fatty acid amides, fatty acid alkanolamides and mixtures of compounds of two or more of these classes of substance.

Suitable ionogenic coemulsifiers are, for example, anionic emulsifiers, such as mono-, di- or triphosphoric esters, soaps (e.g. sodium stearate), fatty alcohol sulfates, but also cationic emulsifiers, such as mono-, di- and trialkylquats and polymeric derivatives thereof.

Available amphoteric emulsifiers are preferably alkylaminoalkylcarboxylic acids, betaines, sulfobetaines and imidazoline derivatives.

It is also possible to use naturally occurring emulsifiers, of these preference being given to beeswax, wool wax, lecithin and sterols.

Fatty alcohol ethoxylates are preferably chosen from the group of ethoxylated stearyl alcohols, cetyl alcohols, cetylstearyl alcohols, in particular polyethylene glycol(13) stearyl ether, polyethylene glycol(14) stearyl ether, polyethylene glycol(15) stearyl ether, polyethylene glycol(16) stearyl ether, polyethylene glycol(17) stearyl ether, polyethylene glycol(18) stearyl ether, polyethylene glycol(19) stearyl ether, polyethylene glycol(20) stearyl ether, polyethylene glycol(12) isostearyl ether, polyethylene glycol(13) isostearyl ether, polyethylene glycol(14) isostearyl ether, polyethylene glycol(15) isostearyl ether, polyethylene glycol(16) isostearyl ether, polyethylene glycol(17) isostearyl ether, polyethylene glycol(18) isostearyl ether, polyethylene glycol(19) isostearyl ether, polyethylene glycol(20) isostearyl ether, polyethylene glycol(13) cetyl ether, polyethylene glycol(14) cetyl ether, polyethylene glycol(15) cetyl ether, polyethylene glycol(16) cetyl ether, polyethylene glycol(17) cetyl ether, polyethylene glycol(18) cetyl ether, polyethylene glycol(19) cetyl ether, polyethylene glycol(20) cetyl ether, polyethylene glycol(13) isocetyl ether, polyethylene glycol(14) isocetyl ether, polyethylene glycol(15) isocetyl ether, polyethylene glycol(16) isocetyl ether, polyethylene glycol(17) isocetyl ether, polyethylene glycol(18) isocetyl ether, polyethylene glycol(19) isocetyl ether, polyethylene glycol(20) isocetyl ether, polyethylene glycol(12) oleyl ether, polyethylene glycol(13) oleyl ether, polyethylene glycol(14) oleyl ether, polyethylene glycol(15) oleyl ether, polyethylene glycol(12) lauryl ether, polyethylene glycol(12) isolauryl ether, polyethylene glycol(13) cetylstearyl ether, polyethylene glycol(14) cetylstearyl ether, polyethylene glycol(15) cetylstearyl ether, polyethylene glycol(16) cetylstearyl ether, polyethylene glycol(17) cetylstearyl ether, polyethylene glycol(18) cetylstearyl ether, polyethylene glycol(19) cetylstearyl ether, polyethylene glycol(20) cetylstearyl ether, polyethylene glycol(20) stearate, polyethylene glycol(21) stearate, polyethylene glycol(22) stearate, polyethylene glycol(23) stearate, polyethylene glycol(24) stearate, polyethylene glycol(25) stearate, polyethylene glycol(12) isostearate, polyethylene glycol(13) isostearate, polyethylene glycol(14) isostearate, polyethylene glycol(15) isostearate, polyethylene glycol(16) isostearate, polyethylene glycol(17) isostearate, polyethylene glycol(18) isostearate, polyethylene glycol(19) isostearate, polyethylene glycol(20) isostearate, polyethylene glycol(21) isostearate, polyethylene glycol(22) isostearate, polyethylene glycol(23) isostearate, polyethylene glycol(24) isostearate, polyethylene glycol(25) isostearate, polyethylene glycol(12) oleate, polyethylene glycol(13) oleate, polyethylene glycol(14) oleate, polyethylene glycol(15) oleate, polyethylene glycol(16) oleate, polyethylene glycol(17) oleate, polyethylene glycol(18) oleate, polyethylene glycol(19) oleate, polyethylene glycol(20) oleate.

In preferred embodiments, polyethylene glycol glycerol fatty acid esters from the group consisting of polyethylene glycol(20) glyceryl laurate, polyethylene glycol(6) glyceryl caprate, polyethylene glycol(20) glyceryl oleate, polyethylene glycol(20) glyceryl isostearate and polyethylene glycol(18) glyceryl oleate/cocoate are used.

Preference is likewise given to using sorbitan esters, in particular polyethylene glycol(20) sorbitan monolaurate, polyethylene glycol(20) sorbitan monostearate, polyethylene glycol(20) sorbitan monoisostearate, polyethylene glycol(20) sorbitan monopalmitate and/or polyethylene glycol(20) sorbitan monooleate.

Advantageous W/O emulsifiers which can be used are the following: fatty alcohols having 8 to 30 carbon atoms, monoglycerol esters of saturated and/or unsaturated, branched and/or unbranched alkanecarboxylic acids with a chain length of from 8 to 24, in particular 18 to 18, carbon atoms, diglycerol esters of saturated and/or unsaturated, branched and/or unbranched alkanecarboxylic acids with a chain length of from 8 to 24, in particular 12 to 18, carbon atoms, monoglycerol ethers of saturated and/or unsaturated, branched and/or unbranched alcohols with a chain length of from 8 to 24, in particular 12 to 18, carbon atoms, diglycerol ethers of saturated and/or unsaturated, branched and/or unbranched alcohols with a chain length of from 8 to 24, in particular 12 to 18, carbon atoms, propylene glycol esters of saturated and/or unsaturated, branched and/or unbranched alkanecarboxylic acids with a chain length from 8 to 24, in particular 12 to 18, carbon atoms, and sorbitan esters of saturated and/or unsaturated, branched and/or unbranched alkanecarboxylic acids with a chain length of from 8 to 24, in particular 12 to 18, carbon atoms.

Particularly advantageous W/O emulsifiers are glyceryl monostearate, glyceryl monoisostearate, glyceryl monomyristate, glyceryl monooleate, glyceryl monolaurate, glyceryl monocaprylate, glyceryl monocaprate, diglyceryl monostearate, diglyceryl monoisostearate, propylene glycol monostearate, propylene glycol monoisostearate, propylene glycol monocaprylate, propylene glycol monolaurate, sorbitan monoisostearate, sorbitan monolaurate, sorbitan monocaprylate, sorbitan monoisooleate, sucrose distearate, cetyl alcohol, stearyl alcohol, arachidyl alcohol, behenyl alcohol, isobehenyl alcohol, selachyl alcohol, chimyl alcohol or polyethylene glycol(2) stearyl ether.

The content of the emulsifier or emulsifiers present in the compositions according to the invention, in addition to the linear organopolysiloxane-polyoxyalkylene copolymer of the formula (I) is preferably 0.1 to 20% by weight, particularly preferably 0.5 to 15% by weight and especially preferably 1 to 10% by weight, based on the finished compositions.

Suitable cationic polymers are preferably the compounds known under the INCI name "Polyquaternium", in particular Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, Polyquaternium-37&mineral oil&PPG trideceth (Salcare^{®} SC95), PVP dimethylaminoethyl methacrylate copolymer, guar hydroxypropyltriammonium chlorides, and calcium alginate and ammonium alginate.

Furthermore, the following may preferably be used: cationic cellulose derivatives; cationic starch; copolymers of diallylammonium salts and acrylamides; quaternized vinylpyrrolidone/vinylimidazole polymers; condensation products of polyglycols and amines; quaternized collagen polypeptides; quaternized wheat polypeptides; polyethyleneimines; cationic silicone polymers, such as, for example, amidomethicones; copolymers of adipic acid and dimethylaminohydroxypropyldiethylenetriamine; polyaminopolyamide and cationic chitin derivatives, such as, for example, chitosan.

The content of cationic polymers in the compositions according to the invention can preferably be in the range from 0.1 to 10% by weight, particularly preferably in the range from 0.2 to 5% by weight and especially preferably in the range from 0.5 to 2.5% by weight.

The desired viscosity of the compositions according to the invention can be adjusted by adding thickeners. Of suitability are preferably cellulose ethers and other cellulose derivatives (e.g. carboxymethylcellulose, hydroxyethylcellulose), gelatin, starch and starch derivatives, sodium alginates, fatty acid polyethylene glycol esters, agar agar, tragacanth or dextrin derivatives, in particular dextrin esters.

The synthetic polymers used are various materials, preferably polyvinyl alcohols, polyacrylamides, polyvinylamides, polysulfonic acids, in particular copolymers based on ammonium salts of acrylamidoalkylsulfonic acids and cyclic N-vinylcarboxamides or cyclic and linear N-vinylcarboxamides and also hydrophobically modified acrylamidoalkylsulfonic acid copolymers, polyacrylic acid, polyacrylic acid derivatives, polyacrylic esters, polyvinylpyrrolidone, polyvinyl methyl ether, polyethylene oxides, copolymers of maleic anhydride and vinyl methyl ether, and various mixtures and copolymers of the abovementioned compounds, including their various salts and esters. These polymers can, if desired, be crosslinked or uncrosslinked.

Thickeners which are particularly suitable especially for oil-based compositions are dextrin esters, for example dextrin palmitate, but also fatty acid soaps, fatty alcohols and silicone waxes, for example alkylmethicones, SilCare^{®} Silicone 41 M65, SilCare^{®} Silicone 41 M70, SilCare^{®} Silicone 41 M80 or SilCare^{®} Silicone 41 M90.

Depending on the intended use, preferred film formers are salts of phenylbenzimidazolesulfonic acid, water-soluble polyurethanes, for example C₁₀-polycarbamylpolyglyceryl esters, polyvinyl alcohol, polyvinylpyrrolidone copolymers, for example vinylpyrrolidone/vinyl acetate copolymer, water-soluble acrylic acid polymers/copolymers or esters or salts thereof, for example partial ester copolymers of acrylic/methacrylic acid and polyethylene glycol ethers of fatty alcohols, such as acrylate/steareth-20 methacrylate copolymer, water-soluble cellulose, for example hydroxymethylcellulose, hydroxyethylcellulose, hydroxypropylcellulose, water-soluble quaterniums, polyquaterniums, carboxyvinyl polymers, such as carbomers and salts thereof, polysaccharides, for example polydextrose and glucan, vinyl acetate/crotonate, available for example under the trade name Aristoflex^{®} A 60 (Clariant), and polymeric amine oxides, for example representatives available under the trade names Diaformer Z-711, 712, 731, 751.

Preferably suitable antimicrobial active ingredients are cetyltrimethylammonium chloride, cetylpyridinium chloride, benzethonium chloride, diisobutylethoxyethyldimethylbenzylammonium chloride, sodium N-laurylsarcosinate, sodium N-palmethylsarcosinate, lauroylsarcosine, N-myristoylglycine, potassium N-laurylsarcosine, trimethylammonium chloride, sodium aluminum chlorohydroxylactate, triethyl citrate, tricetylmethylammonium chloride, 2,4,4'-trichloro-2'-hydroxydiphenyl ether (triclosan), phenoxyethanol, 1,5-pentanediol, 1,6-hexanediol, 3,4,4'-trichlorocarbanilide (triclocarban), diaminoalkylamide, for example L-lysinehexadecylamide, citrate heavy metal salts, salicylates, piroctose, in particular zinc salts, pyrithiones and heavy metal salts thereof, in particular zinc pyrithione, zinc phenol sulfate, farnesol and combinations of these active substances.

The compositions according to the invention comprise the antimicrobial agents preferably in amounts up to 50% by weight, particularly preferably in amounts of from 0.01 to 10% by weight and especially preferably in amounts of from 0.1 to 10% by weight.

Preferred astringents are oxides, preferably magnesium oxide, aluminum oxide, titanium dioxide, zirconium dioxide and zinc oxide, oxide hydrates, preferably aluminum oxide hydrate (boehmite) and hydroxides, preferably of calcium, magnesium, aluminum, titanium, zirconium or zinc.

The compositions according to the invention comprise the astringent active ingredients preferably in amounts of from 0 to 50% by weight, particularly preferably in amounts of from 0.01 to 10% by weight and especially preferably in amounts of from 0.1 to 10% by weight.

Advantageous compositions according to the invention comprise one or more antioxidants. Favorable, but nevertheless optional, antioxidants which can be used are all antioxidants which are customary or suitable for cosmetic and/or pharmaceutical application.

The antioxidants are advantageously chosen from the group consisting of amino acids (e.g. glycine, histidine, tyrosine, tryptophan) and derivatives thereof, imidazoles (e.g. urocanic acid) and derivatives thereof, peptides such as D,L-carnosine, D-carnosine, L-carnosine and derivatives thereof (e.g. anserine), carotenoids, carotenes (e.g. α-carotene, β-carotene, lycopene) and derivatives thereof, chlorogenic acid and derivatives thereof, lipoic acid and derivatives thereof (e.g. dihydrolipoic acid), aurothioglucose, propylthiouracil and other thiols (e.g. thioredoxin, glutathione, cysteine, cystine, cystamine and the glycosyl, N-acetyl, methyl, ethyl, propyl, amyl, butyl and lauryl, palmitoyl, oleyl, γ-linoleyl, cholesteryl and glyceryl esters thereof) and salts thereof, dilauryl thiodipropionate, distearyl thiodipropionate, thiodipropionic acid and derivatives thereof (esters, ethers, peptides, lipids, nucleotides, nucleosides and salts), and sulfoximine compounds (e.g. buthionine sulfoximines, homocysteine sulfoximine, buthionine sulfones, penta-, hexa-, heptathionine sulfoximine) in very low tolerated doses (e.g. pmol/kg), and also (metal) chelating agents (e.g. α-hydroxyfatty acids, palmitic acid, phytic acid, lactoferrin), α-hydroxy acids (e.g. citric acid, lactic acid, malic acid), humic acid, bile acid, bile extracts, bilirubin, biliverdin, EDTA, EGTA and derivatives thereof, unsaturated fatty acids and derivatives thereof (e.g. γ-linolenic acid, linoleic acid, oleic acid), folic acid and derivatives thereof, ubiquinone and ubiquinol and derivatives thereof, vitamin C and derivatives (e.g. ascorbyl palmitate, Mg ascorbyl phosphate, ascorbyl acetate), tocopherols and derivatives (e.g. vitamin E acetate), vitamin A and derivatives (e.g. vitamin A palmitate), and coniferyl benzoate of benzoin resin, rutinic acid and derivatives thereof, α-glycosylrutin, ferulic acid, furfurylideneglucitol, carnosine, butylhydroxytoluene, butylhydroxyanisole, nordihydroguaiacic acid, nordihydroguaiaretic acid, trihydroxybutyrophenone, uric acid and derivatives thereof, mannose and derivatives thereof, zinc and derivatives thereof (e.g. ZnO, ZnSO₄), selenium and derivatives thereof (e.g. selenomethionine), stilbenes and derivatives thereof (e.g. stilbene oxide, trans-stilbene oxide), superoxide dismutase and the derivatives (salts, esters, ethers, sugars, nucleotides, nucleosides, peptides and lipids) of these specified substances which are suitable according to the invention.

For the purposes of the present invention, water-soluble antioxidants can be used particularly advantageously.

The antioxidants can protect the skin and the hair against oxidative stress. Preferred antioxidants here are vitamin E and derivatives thereof, and vitamin A and derivatives thereof.

The amount of antioxidants (one or more compounds) in the compositions according to the invention is preferably 0.001 to 30% by weight, particularly preferably 0.05 to 20% by weight and, in particular 1 to 10% by weight, based on the total weight of the compositions.

If vitamin E and/or derivatives thereof are the antioxidant or the antioxidants, it is advantageous to choose their particular concentrations from the range from 0.001 to 10% by weight, based on the total weight of the compositions.

If vitamin A and/or derivatives thereof, or carotenes or derivatives thereof are the antioxidant or the antioxidants, it is advantageous to choose their particular concentrations from the range from 0.001 to 10% by weight, based on the total weight of the compositions.
In a particularly preferred embodiment of the invention, the cosmetic or pharmaceutical compositions according to the invention comprise antioxidants chosen from superoxide dismutase, tocopherol (vitamin E) and ascorbic acid (vitamin C).

In a further preferred embodiment, the compositions according to the invention comprise one or more UV filters.

Suitable UV filters are preferably 4-aminobenzoic acid; 3-(4'-trimethylammonium)-benzylidenebornan-2-one methylsulfate; 3,3,5-trimethyl cyclohexylsalicylate; 2-hydroxy-4-methoxybenzophenone; 2-phenylbenzimidazole-5-sulfonic acid and its potassium, sodium and triethanolamine salts; 3,3'-(1,4-phenylenedimethine)bis-(7,7-dimethyl-2-oxobicyclo[2.2.1]heptane-1-methanesulfonic acid and its salts; 1-(4-tert-butylphenyl)-3-(4-methoxyphenyl)propane-1,3-dione, 3-(4'-sulfo)-benzylidenebornan-2-one and its salts; 2-ethylhexyl 2-cyano-3,3-diphenylacrylate; polymers of N-[2(and 4)-(2-oxoborn-3-ylidenemethyl)benzyl]acrylamide; 2-ethylhexyl 4-methoxycinnamate; ethoxylated ethyl 4-aminobenzoate; isoamyl 4-methoxycinnamate; 2,4,6-tris[p-(2-ethylhexyloxycarbonyl)anilino]-1,3,5-triazine; 2-(2H-benzotriazol-2-yl)-4-methyl-6-(2-methyl-3-(1,3,3,3-tetramethyl-1-(trimethylsilyloxy)-disiloxanyl)propyl)phenol; bis(2-ethylhexyl) 4,4'-[(6-[4-((1,1-dimethylethyl)aminocarbonyl)phenylamino]-1,3,5-triazin-2,4-yl)diimino]bisbenzoate; 3-(4'-methylbenzylidene)-D,L-camphor; 3-benzylidenecamphor; 2-ethylhexyl salicylate; 2-ethylhexyl 4-dimethylaminobenzoate; hydroxy-4-methoxybenzophenone-5-sulfonic acid (sulisobenzonum) and the sodium salt; and/or 4-isopropylbenzyl salicylate.

Pigments/micropigments which may be used are preferably microfine titanium dioxide, mica-titanium dioxide, iron oxides, mica-iron oxide, zinc oxide, silicon oxides, ultramarine blue, chromium oxides.

Suitable gelling agents are all surface-active substances which, dissolved in the liquid phase, form a network structure and thus consolidate the liquid phase. Suitable gelling agents are specified, for example, in WO 98/58625.

Preferred gelling agents are metal salts of fatty acids, preferably with 12 to 22 carbon atoms, for example sodium stearate, sodium palmitate, sodium laurate, sodium arachidate, sodium behenate, potassium stearate, potassium palmitate, sodium myristate, aluminum monostearate, hydroxyfatty acids, for example 12-hydroxystearic acid, 16-hydroxyhexadecanoyl acid; fatty acid amides; fatty acid alkanolamides; dibenzalsorbitol and alcoholic polyamides and polyacrylamides or mixtures thereof.

Preferably, the compositions according to the invention comprise 0.01 to 20% by weight, particularly preferably 0.1 to 10% by weight, especially preferably 1 to 8% by weight and very particularly preferably 3 to 7% by weight, of gelling agents, based on the total weight of the compositions.

Further additives may be silicone compounds, preferably dimethylpolysiloxane, methylphenylpolysiloxanes, cyclic silicones, and amino-, fatty acid-, alcohol-, polyether-, epoxy-, fluorine- and/or alkyl-modified silicone compounds, for example alkylsilicones SiICare^{®} Silicone 41M10, SiICare^{®} Silicone 41 M15, SiICare^{®} Silicone 41 M20, SiICare^{®} Silicone 41 M30 (Clariant), alkyltrimethicones SiICare^{®} 31 M30, SiICare^{®} 31 M40, SiICare^{®} 31 M 50, SiICare^{®} 31 M 60 (Clariant), phenyltrimethicones SiICare^{®} 15M30, SiICare^{®} 15M40, SiICare^{®} 15M50, SiICare^{®} 15M60 (Clariant), polyalkylarylsiloxanes and polyethersiloxane copolymers.

The compositions according to the invention can comprise the abovementioned silicone compounds preferably in the amounts by weight of from 0.1 to 20% by weight, particularly preferably 0.2 to 15% by weight and especially preferably 0.5 to 10% by weight, based on the finished compositions.

Suitable carrier materials are preferably vegetable oils, natural and hydrogenated oils, waxes, fats, water, alcohols, polyols, glycerol, glycerides, liquid paraffins, liquid fatty alcohols, sterol, polyethylene glycols, cellulose and cellulose derivatives.

Fungicidal active ingredients which may be used are preferably ketoconazole, oxiconazole, terbinafin, bifonazole, butoconazole, cloconazole, clotrimazole, econazole, enilconazole, fenticonazole, isoconazole, miconazole, sulconazole, tioconazole, fluconazole, itraconazole, terconazole and naftifine, Zn pyrithione and Octopirox^{®} in the amounts by weight of from 0.05 to 5% by weight, preferably 0.1 to 3% by weight and particularly preferably 0.2 to 2% by weight, based on the finished compositions.

The compositions according to the invention can advantageously be mixed with conventional ceramides, pseudoceramides, fatty acid N-alkylpolyhydroxyalkylamides, cholesterol, cholesterol fatty acid esters, fatty acids, triglycerides, cerebrosides, phospholipids and similar substances.

As pearlescence-imparting compounds, preference is given to fatty acid monoalkanolamides, fatty acid dialkanolamides, monoesters or diesters of alkylene glycol, in particular of ethylene glycol and/or propylene glycol or oligomers thereof with higher fatty acids, e.g. palmitic acid, stearic acid or behenic acid or mixtures thereof, monoesters or diesters of alkylene glycols with fatty acids, fatty acids and metal salts thereof, monoesters or polyesters of glycerol with carboxylic acids and ketosulfones of various types. In the compositions according to the invention, the pearlescence-imparting component is particularly preferably ethylene glycol distearate and polyethylene glycol distearate with 3 glycol units.

The moisturizing substances available are preferably isopropyl palmitate, glycerol and/or sorbitol, which are preferably used in the amounts by weight of from 0.1 to 50%.

Superfatting agents which may be used are preferably lanolin and lecithin, nonethoxylated and polyethoxylated or acylated lanolin and lecithin derivatives, polyol fatty acid esters, mono-, di- and triglycerides and/or fatty acid alkanolamides.

Suitable preservatives are preferably phenoxyethanol, parabens, pentanediol or sorbic acid, although silver ions and other customary preservatives have also proven suitable. They are preferably used in the amounts by weight of from 0.001 to 5% by weight, particularly preferably from 0.01 to 3% by weight and especially preferably from 0.1 to 2% by weight, based on the finished compositions.

Dyes which can be used are the substances approved and suitable for cosmetic and pharmaceutical purposes.

Fragrance and/or perfume oils which may be used are individual odorant compounds, e.g. the synthetic products of the ester, ether, aldehyde, ketone, alcohol and hydrocarbon type. Odorant compounds of the ester type are, for example, benzyl acetate, phenoxyethyl isobutyrate, p-tert-butylcyclohexyl acetate, linalyl acetate, dimethylbenzylcarbinyl acetate, phenylethyl acetate, linalyl benzoate, benzyl formate, ethyl methylphenylglycinate, allyl cyclohexylpropionate, styrallyl propionate and benzyl salicylate. The ethers include, for example, benzyl ethyl ether, and the aldehydes include, for example, the linear alkanals having 8 to 18 carbon atoms, citral, citronellal, citronellyloxyacetaldehyde, cyclamen aldehyde, hydroxycitronellal, lilial and bourgeonal, and the ketones include, for example, the ionones, alpha-isomethylionone and methyl cedryl ketone, and the alcohols include anethol, citronellol, eugenol, geraniol, linalool, phenylethyl alcohol and terpineol, and the hydrocarbons include primarily the terpenes and balsams. Preference is given to using mixtures of different odorants which together produce a pleasant scent note.

Perfume oils can also comprise natural odorant mixtures, as are accessible from vegetable or animal sources, e.g. pine, citrus, jasmine, lily, rose or ylang ylang oil. Essential oils of lower volatility, which are mostly used as aroma components, are also suitable as perfume oils, e.g. sage oil, camomile oil, clove oil, melissa oil, mint oil, cinnamon leaf oil, linden blossom oil, juniper berry oil, vetiver oil, olibanum oil, galbanum oil and ladanum oil.

The acids or alkalis used for adjusting the pH are preferably mineral acids, for example HCl, inorganic bases, for example NaOH, KOH and organic acids, preferably citric acid.

The compositions according to the invention are preferably adjusted to a pH in the range from 2 to 12, particularly preferably to a pH from 3 to 8 and especially preferably to a pH from 4 to 8.

The linear organopolysiloxane-polyoxyalkylene copolymers of the formula (I) are advantageously suitable for the cleansing and care of the hair and the skin. The present invention therefore also further provides the use of one or more linear organopolysiloxane-polyoxyalkylene copolymers of the formula (I), preferably in cosmetic or dermatological compositions, for the cleansing and care of the hair and the skin.

Furthermore, the linear organopolysiloxane-polyoxyalkylene copolymers of the formula (I) are suitable as emulsifier in emulsions, preferably in O/W or in W/O emulsions. The present invention thus also further provides the use of one or more linear organopolysiloxane-polyoxyalkylene copolymers of the formula (I) as emulsifier in emulsions, preferably in O/W emulsions or in W/O emulsions.

In addition, the linear organopolysiloxane-polyoxyalkylene copolymers of the formula (I) are advantageously suitable as conditioners. The present invention therefore also further provides the use of one or more linear organopolysiloxane-polyoxyalkylene copolymers of the formula (I), preferably in cosmetic or dermatological compositions, as conditioners.

Furthermore, the linear organopolysiloxane-polyoxyalkylene copolymers of the formula (I) are advantageously suitable as emulsifier in emulsions and simultaneously as conditioners, preferably in O/W or W/O emulsions. The present invention thus also further provides the use of one or more linear organopolysiloxane-polyoxyalkylene copolymers of the formula (I) as emulsifier and at the same time as conditioner in emulsions, preferably in O/W or W/O emulsions, in particular in those emulsions which are in the form of cosmetic or dermatological compositions.

The examples and applications below are intended to illustrate the invention in more detail without, however, limiting it thereto (all of the percentages given are percentages by weight).

### Examples

### Example 1: Preparation of SilCare^{®} Silicone ABA 250, low molecular weight

52 g of Genapol^{®} TA 250 and 50 g of tetrahydrofuran were heated to 70°C, then 50 µl of Ashby's catalyst (Pt catalyst) and 4 g of dimethylpolysiloxane MHD₂₅MH were added. A further 31.8 g of MHD₂₅MH were metered in over the course of 30 minutes and then heated at 90°C for 10 hours. The course of the reaction was monitored using IR spectroscopy by reference to the Si-H stretch vibration at 2100 cm⁻¹. The reaction solution was cooled to room temperature, 50 g of hexamethyldisiloxane were added and the reaction mixture was heated to 110°C and the pressure was slowly reduced to 0.5 mmHg.

### Example 2: Preparation of SilCare^{®} Silicone ABA 250, high molecular weight

523 g of MHD₈₀MH, 226 g of Genapol^{®} TA 250, 215 g of tetrahydrofuran, 0.9 g of a 3% by weight strength solution of H₂PtCl₆ in 1,3,5,7-tetramethyl-1,3,5,7-tetravinylcyclotrisiloxane were heated at 70°C to 90°C for 10 hours. Then, THF was stripped off at 140°C and a pressure of 0.5 mmHg. The reaction solution was cooled to room temperature, 80 g of hexamethyldisiloxane was added and dissolved. Following filtration, hexamethyldisiloxane was distilled off at reduced pressure.

### Example 3: Preparation of SiICare^{®} Silicone ABA 080, low molecular weight

30 g of Genapol^{®} TA 080 (20 mol% in excess) and 30 g of tetrahydrofuran were heated to 70°C, then 60 µl of Ashby's catalyst and 4 g of dimethylpolysiloxane MHD25MH were added. A further 32 g of MHD25MH was metered in over the course of 30 minutes and then heated at 90°C for 5 hours. The course of the reaction was monitored using IR spectroscopy by reference to disappearance of the Si-H stretch vibration at 2100 cm⁻¹. The reaction solution was cooled to room temperature, 40 g of hexamethyldisiloxane was added and the reaction mixture heated to 110°C and the pressure slowly reduced to 0.5 mmHg to strip off THF solvent as well as hexamethyldisiloxane. 62 g of Product was isolated.

### Example 4: Preparation of SilCare^{®} Silicone ABA 080, low molecular weight in IPA solvent

24.5 g of Genapol^{®} TA 080 (0 mo)% in excess) and 80 ml of isopropanol were heated to 80°C then 30 µl of Ashby's catalyst was added. 36 g of dimethylpolysiloxane MHD25MH were gradually added into the reactor over the course of 30 minutes while maintaining the reaction temperature at 80°C. The reaction was continued for another 16 hours at 80°C. The course of the reaction was monitored using IR spectroscopy by reference to disappearance of the Si-H stretch vibration at 2100 cm⁻¹. Isopropanol solvent was removed at 110°C under vacuum (the pressure was slowly reduced to 0.5 mmHg) to isolate the product.

### Application examples

### Example 5: Cream gel

| | | | % by wt. |
|---|---|---|---|
| A | Mineral oil, low viscosity | | 7.00 |
| | SiICare^{®} 15 M50 | Clariant | 5.00 |
| | ABA Silicone Copolyol (Ex.1) | Clariant | 1.00 |
| B | Aristoflex^{®} AVC | Clariant | 1.00 |
| C | Water | | ad 100 |
| | Glycerol | | 8.00 |
| | Preservative | | q.s. |
| D | Fragrance | | 0.30 |

### Preparation method:

- I: Mixing of A and B
- II: Stirring of C into I, then addition of D
- III: Homogenization

### Example 6: Cream rinse

| | | | % by wt. |
|---|---|---|---|
| A | Hostacerin^{®} DGI | Clariant | 1.50 |
| | Cetyl alcohol | | 3.00 |
| | ABA Silicone Copolyol (Ex. 2) | Clariant | 1.00 |
| B | Genamin^{®} CTAC | Clariant | 3.30 |
| | Water | | ad 100 |
| | Preservative | | q.s. |
| C | Fragrance | | 0.30 |

### Preparation method:

- I: Melting of A at about 75°C
- II: Heating of B to about 75°C
- III: Addition of II to I with stirring and further stirring to 30°C
- IV: Addition of C to III at 30°C
- V: Adjustment to pH 4.0 with citric acid

### Example 7: Antiacne gel

| | | | % by wt. |
|---|---|---|---|
| A | Octopirox^{®} | Clariant | 0.10 |
| B | Ethanol | Clariant | 25.00 |
| | Propylene glycol | | 20.00 |
| | ABA Silicone Copolyol (Ex. 2) | Clariant | 1.00 |
| C | Perfume | | 0.20 |
| | Nipaguard^{®} CMB | Clariant | 0.10 |
| D | Aristoflex^{®} HMB | Clariant | 1.30 |
| E | Allantoin^{®} | Clariant | 0.10 |
| F | Water | | ad 100 |

### Preparation method:

- I: Dissolution of A in B
- II: Addition of C to I
- III: Stirring of D into II

- IV: Dissolution of E in heated water
- V: Addition of IV to III with stirring

### Example 8: Hair shampoo

| | | % by wt. |
|---|---|---|
| A | Genapol^{®} LRO liq. | 48.10 |
| B | ABA Silicone Copolyol (Ex. 1) | 1.00 |
| | Fragrance | 0.30 |
| | Water | ad 100 |
| | Genagen^{®} CAB | 6.70 |
| | Dye solution | q.s. |
| | Preservative | q.s. |
| C | Sodium chloride | 1.50 |

### Preparation:

- I: Successive addition of components B to A with stirring
- II: Adjust to pH 5 to 7 with citric acid
- III: Adjustment of the viscosity with C

### Example 9: Hair shampoo test formulation

| | | % by wt. |
|---|---|---|
| A | Genapol^{®} LRO liq. | 48.10 |
| B | ABA Silicone Copolyol (Ex. 2) | 1.00 |
| | Fragrance | 0.30 |
| | Water | ad 100 |
| | Genagen^{®} CAB | 6.70 |
| | Dye solution | q.s. |
| | Preservative | q.s. |
| C | Sodium chloride | 1.50 |

### Preparation:

- I: Successive addition of components B to A with stirring
- II: Adjust to pH 5 to 7 with citric acid
- III: Adjustment of the viscosity with C

### Example 10: Moisture cream gel

| | | | % by wt. |
|---|---|---|---|
| A | Mineral oil, low viscosity | | 7.00 |
| | SiICare^{®} 15 M50 | Clariant | 5.00 |
| B | Aristoflex^{®} AVC | Clariant | 1.00 |
| C | Water | | ad 100 |
| | Glycerol | | 8.00 |
| | ABA Silicone Copolyol (Ex. 1) | Clariant | 1.00 |
| | Preservative | | q.s. |
| D | Fragrance | | 0.30 |

### Preparation method:

- I: Mixing of A and B
- II: Stirring of C into I, then addition of D
- III: Homogenization

### Example 11: Skincare oil

| | | | % by wt. |
|---|---|---|---|
| A | SilCare^{®} Silicone 31 M50 | Clariant | 40.00 |
| | SilCare^{®} Silicone 41M15 | Clariant | 20.00 |
| | Caprylic/Capric Triglyceride | | 38.60 |
| | ABA Silicone Copolyol (Ex. 2) | Clariant | 1.00 |
| | SilCare^{®} Silicone 1 M75 | Clariant | 0.40 |

### Preparation method:

- I: Mixing of component A

### Example 12: Hair ends care

| | | | % by wt. |
|---|---|---|---|
| A | Water | | 50.00 |
| B | Tylose^{®} H 100000 G4 | | 1.00 |
| C | Water | | ad 100 |
| D | Genamin^{®} PDAC | Clariant | 2.50 |
| | Glycerol | | 2.00 |
| | ABA Silicone Copolyol (Ex. 1) | Clariant | 1.00 |
| E | Citric acid | | q.s. |

### Preparation method:

- I: Swell B in A
- II: Successive dissolution of the individual components of D in C
- III: Addition of II to I
- IV: Adjustment of the pH with E

### Example 13: Antiperspirant

| | | | % by wt. |
|---|---|---|---|
| A | Locron^{®} L | Clariant | 10.00 |
| | Ethanol | | 50.00 |
| | Farnesol | | 0.50 |
| | Fragrance | | 0.20 |
| | Water | | ad 100 |
| | Extrapon Avocado special | | 0.50 |
| | ABA Silicone Copolyol (Ex. 2) | Clariant | 1.00 |

### Preparation method:

Mixing of components A

### Example 14: Deodorant

| | | | % by wt. |
|---|---|---|---|
| A | Octopirox^{®} | Clariant | 0.30 |
| B | Ethanol | Clariant | 70.00 |
| C | Perfume | | 0.50 |
| | Softigen^{®} 767 | | 0.50 |
| D | Allantoin | Clariant | 0.10 |
| | ABA Silicone Copolyol (Ex. 4) | Clariant | 1.00 |
| E | Water | | ad 100 |
| F | Citric acid | | q.s. |

### Preparation method:

- I: Mixing of A and B
- II: Addition of C to I
- III: Dissolution of D in warm E, addition of II
- IV: Adjustment of the pH with F

### Example 15: Sensory properties application example

Standard shampoo (without silicone) as benchmark

| | | % by wt. |
|---|---|---|
| A | Genapol^{®} LRO liq. | 48.10 |
| B | Fragrance | 0.30 |
| | Water | ad 100 |
| | Genagen^{®} CAB | 6.70 |
| | Dye solution | q.s. |
| | Preservative | q.s. |
| C | Sodium chloride | 1.50 |

### Preparation:

- I: Successive addition of the components B to A with stirring
- II: Adjust to pH 6 to 7 with citric acid
- III: Adjustment of the viscosity with C

Blonde, bleached, European hair tresses were each treated under defined conditions with the noninventive standard shampoo as comparison and the Si-copolyol-containing compositions according to the invention of examples 8 and 9. The sensory assessment took place in a panel test (10 persons).

The parameters foam stability, wet combability, dry combability and feel of the test formulations are listed below in table 1 (average from all test persons).

**Table: 1 Comparison of the standard shampoo from example 15 with the compositions according to the invention from examples 8 and 9 with regard to the parameters foam stability, wet combability, dry combability and feel**

| Formulation | Foam stability | Wet combability | Dry combability | Feel |
|---|---|---|---|---|
| Benchmark (comparison) | ○ | ○ | ○ | ○ |
| Example 8 (invention) | + | o | + | +++ |
| Example 9 (invention) | + | + | + | ++ |

| | | | | |
|---|---|---|---|---|
| o: standard +: slightly improved compared with standard ++: improved compared with standard +++: significantly improved compared with standard | | | | |

The results in table 1 show that in particular the foam stability, the dry combability and very particularly the feel is improved in the presence of the linear alkoxylated polyorganosiloxanes of the formula (I) according to the invention. A significantly improved conditioning effect for the hair is achieved.

In addition, the compositions according to the invention produce a pleasant foam feel and bring about a very finely divided foam.

| | | % by wt. |
|---|---|---|
| A | Genapol^{®} LRO liq. | 48.10 |
| B | Fragrance | 0.30 |
| | Water | ad 100 |
| | Genagen^{®} CAB | 6.70 |
| | Dye solution | q.s. |
| | Preservative | q.s. |

### Example 16: Sunscreen Cream Gel

| | | | % by wt. |
|---|---|---|---|
| A | Eusolex^{®} 232 | | 8.00 |
| B | Water | | ad 100 |
| C | Aristoflex^{®} HMB | Clariant | 2.00 |
| D | Tegosoft TN | | 5.00 |
| | SilCare^{®} Silicone 15M50 | Clariant | 4.00 |
| | Eusolex^{®} 9020 | | 3.00 |
| | Velsan^{®} D8P | | 3.50 |
| E | Preservative | | q.s. |
| | Genapol^{®} LA 070 | | 0.70 |
| | ABA Silicone Copolyol (Ex. 1) | Clariant | 1.00 |
| | Fragrance | | q.s. |

### Preparation:

- I: Mix A and B and neutralize it (adjust the pH to approx. 7.3).
- II: Add C and stir until a homogeneous gel has been obtained.
- III: Mix the components of D, dissolve them by slightly heating and add them to II.
- IV: Finally add E to III.

### Example 17: Sunscreen Cream

| | | | % by wt. |
|---|---|---|---|
| A | Eusolex^{®} 9020 | | 3.00 |
| | Eusolex^{®} 2292 | | 7.50 |
| | Eusolex^{®} OS | | 5.00 |
| | Eusolex^{®} OCR | | 9.50 |
| | Neo Heliopan^{®} BB | | 6.00 |
| | Velsan^{®} P8-3 | Clariant | 10.00 |
| | SiICare^{®} Silicone 31 M30 | Clariant | 4.00 |
| | SilCare Silicone 1 M71 | Clariant | 1.00 |
| | ABA Silicone Copolyol (Ex. 1) | Clariant | 0.50 |
| B | Aristoflex^{®} AVC | | 1.25 |
| C | Water | | ad 100 |
| | Hostaphat^{®} CK 100 | Clariant | 1.00 |
| | Sodium Hydroxide (10% aqueous) | | 2.00 |
| D | Fragrance | Clariant | 0.40 |
| | Preservatives | Clariant | 0.50 |

### Preparation:

- I: Melt A at 80°C, then add B.
- II: Melt C at 80°C.
- III: Mix Stir II into I and stir until cool.
- IV: At 35°C add D to III.
- V: Finally homogenize the emulsion.

### Example 18: Sunscreen Cream Gel

| | | | % by wt. |
|---|---|---|---|
| A | Jojoba Oil | | 8.00 |
| | Velsan^{®} CCT | Clariant | 5.00 |
| | Isopropyl Myristate | | 3.00 |
| | UV Titan M 262 | | 8.00 |
| | ABA Silicone Copolyol (Ex. 1) | Clariant | 0.75 |
| B | Aristoflex^{®} HMB | Clariant | 0.80 |
| C | Water | | ad 100 |
| | Preservatives | | 0.50 |
| | Fragrance | | q.s. |

### Preparation:

- I: Disperse the components of A.
- II: Add B to I.
- II: Mix the components of C, add them to II and stir until a homogenous cream gel has been obtained.

### Example 19: Sun Blocker Cream

| | | | % by wt. |
|---|---|---|---|
| A | Hostaphat^{®} CS 120 | Clariant | 3.00 |
| | Tegin^{®} M | | 2.50 |
| | Stearic acid | | 2.00 |
| | Cetyl Alcohol | | 1.00 |
| | Albil^{®} 100 | | 2.00 |
| | Minearl Oil, low viscosity | | 3.00 |
| | Cetiol^{®} 868 | | 3.00 |
| | Velsan^{®} CCT | Clariant | 3.00 |
| | Eusolex^{®} 6300 | | 4.00 |
| | ABA Silicone Copolyol (Ex. 1) | Clariant | 2.00 |
| B | Permulen^{®} TR 1 | | 0.20 |
| | UV-Titan M210 | | 5.00 |
| C | Hostaphat^{®} CLG | Clariant | 0.60 |
| | Eusolex^{®} 232 | | 4.00 |
| | Tris (hydroxymethyl) aminomethan | | 2.21 |
| | Allantoin | | 0.20 |
| | Glycerin | | 5.00 |
| | Preservative | | q.s. |
| | Water | | ad 100 |
| D | Fragrance | | 0.30 |

### Preparation:

- I: Melt A at 80°C, then add the components of B.
- II: Homogenize I (Ultra Turrax).
- III: Heat C to 80°C.
- IV: Stir II into I.
- V: Stir until cool.
- VI: Add D to IV at 35°C.
- VII: Finally homogenize the emulsion.

### Chemical designation of the commercial products used

| | | |
|---|---|---|
| Albil^{®} 100 | (Degussa) | Dimethicone |
| Allantoin^{®} | (Clariant) | 2,5-Dioxo-4-imidazolidinylurea |
| Aristoflex^{®} AVC | (Clariant) | Ammoniumacryloyldimethyltaurate/ |
| | | NVP copolymer |
| | | (NVP: N-vinylpyrrolidone) |
| Aristoflex^{®} HMB | (Clariant) | Ammoniumacryloyldimethyltaurate/ |
| | | Beheneth-25 methacrylate |
| | | polymer |
| Aristoflex^{®} PEA 70 | (Clariant) | Polypropylene terephthalate |
| Cetiol^{®} 868 | | Ethylhexyl Stearate |
| Cetiol^{®} V | (Cognis) | Decyl oleate |
| | | Cetyl Alcohol |
| Diaformer Z-751 | | Lauryl/stearyl acrylate, |
| | | ethyleneamine oxide, |
| | | methacrylate |
| | | copolymer |
| Emulsogen^{®} DTC, acid | (Clariant) | Trideceth-7 carboxylic acid |
| Emulsogen^{®} HCO 040 | (Clariant) | PEG-40 hydrogenated castor oil |
| Eusolex^{®} 232 | | Phenylbenzimidazole Sulfonic Acid |
| Eusolex^{®} 9020 | | Butyl Methoxydibenzoylmethane |
| Eusolex^{®} 2292 | | Ethylhexyl Methoxycinnamate |
| Eusolex^{®} 2292 | | Octocrylene |
| Eusolex^{®} 6300 | | 4-Methylbenzylidene Camphor |
| Eusolex^{®} OS | | Ethylhexyl Salicylate |
| Extrapon Avocado special | | Water/ethoxydiglycol/propylene |
| | | glycol/butylene glycol/Persea |
| | | gratissima extract |
| Genagen^{®} CAB | (Clariant) | Cocoamidopropylbetaine |
| Genagen^{®} KB | (Clariant) | Cocobetaine |
| Genagen^{®} LAA (Clariant) | | Sodium lauroamphoacetate |
| Genamin^{®} CTAC | (Clariant) | Cetrimonium chloride |
| Genamin^{®} KSL | (Clariant) | PEG-5 stearylammonium lactate |
| Genamin^{®} PDAC | (Clariant) | Polyquaternium-6 |
| Genapol^{®} C100 | (Clariant) | Coceth-10 |
| Genapol^{®} PDB | (Clariant) | Glycol distearate/Laureth-4/ |
| | | cocoamidopropylbetaine |
| Genapol^{®} LA 070 | (Clariant) | Laureth-7 |
| Genapol^{®} LRO liq. | (Clariant) | Sodium laureth sulfate |
| Genapol^{®} TA 080 | (Clariant) | Ceteareth-8 allyl ether |
| Genapol^{®} TA 250 | (Clariant) | Ceteareth-25 allyl ether |
| Genapol^{®} T 500 P | (Clariant) | Ceteareth-50 |
| Glucamat DOE-120 | | PEG-120 methylglucose |
| | | Dioleate Glycerin |
| Hostaphat^{®} CK 100 | (Clariant) | Potassium Cetyl Phosphate |
| Hostaphat^{®} CS 120 | (Clariant) | Stearyl Phosphate |
| Hostacerin^{®} DGI | (Clariant) | Polyglyceryl-2 sesquiisostearate |
| Hostapon^{®} CLG | (Clariant) | Sodium Lauroyl Glutamate |
| Hostapon^{®} KCG | (Clariant) | Sodium cocoyl glutamate |
| Jojoba Oil | | |
| Isopropyl Myristate | | |
| Locron^{®} L | (Clariant) | Aluminum chlorohydrate |
| Lunacera^{®} M | (H.B. Fuller) | Microcrystalline wax |
| | | Mineral Oil, low viscosity |
| MHD₂₅ MH | | Dimethylpolysiloxysilane (25 Si-O) |
| MHD₈₀ MH | | Dimethylpolysiloxysilane (80 Si-O) |
| Neo Heliopan^{®} BB | | Benzophenone-3 |
| Nipaguard^{®} CMB | (Clariant) | Triethylene glycol/benzyl alcohol/ |
| | | propylene glycol/ |
| | | chloromethylisothiazolinone/ |
| | | methylisothiazolinone |
| Nipaguard^{®} DCB | (Clariant) | Phenoxyethanol, methyl- |
| | | dibromoglutaronitrile |
| Nipaguard^{®} DMDMH | (Clariant) | DMDM hydantoin |
| Nipaguard^{®} PDU | (Clariant) | Propylene glycol/diazolidinylurea/ |
| | | methylparaben/propylparaben |
| Octopirox^{®} | (Clariant) | Piroctone olamine |
| Permulen^{®} TR1 | | Acrylates/C10-30 Alkylacrylate |
| | | Polymer |
| Phenonip^{®} | (Clariant) | Phenoxyethanol/methyl/ethyl/ |
| | | butyl/propyl/isobutylparaben |
| SilCare^{®} Silicone 1 M71 | (Clariant) | Stearoxytrimethylsilane |
| SilCare^{®} Silicone 1 M75 | (Clariant) | Retinoxytrimethylsilane |
| SilCare^{®} Silicone 15M50 | (Clariant) | Phenyltrimethicone |
| SiICare^{®} Silicone 31 M30 | (Clariant) | Caprylyl Trimethicone |
| SiICare^{®} Silicone 31 M50 | (Clariant) | Caprylyltrimethicone |
| SiICare^{®} Silicone 41 M15 | (Clariant) | Caprylylmethicone |
| SiICare^{®} Silicone SEA | (Clariant) | Trideceth-9 PG amodimethicone |
| | | and Trideceth-9 |
| Softigen^{®} 767 | (Sasol) | PEG-6 caprylic/capric glyceride |
| | | Stearic Acid |
| Tegin M | | Glyceryl Stearate |
| Tegosoft TN | | C₁₂-C₁₅ Alkylbenzoat |
| Tris (hydroxymethyl) aminomethan | | Tromethamine |
| Tylose^{®} H 100000 G4 | (ShinEtsu) | Hydroxyethylcellulose |
| UV Titan M 210 | | Ultrafine Titanium Dioxide |
| UV Titan M 262 | | Titanium Dioxide |
| Velsan CCT | (Clariant) | Capric/Caprylic Triglyceride |
| Velsan D8P-3 | (Clariant) | Isopropyl PPG-2 Isedeceth-7 |
| | | Carboxylate |
| Velsan P8-3 | (Clariant) | Isopropyl C₁₂₋₁₅ Pareth-9 |
| | | Carboxylate |

## Claims

1. A linear organopolysiloxane-polyoxyalkylene copolymer of the formula (I) 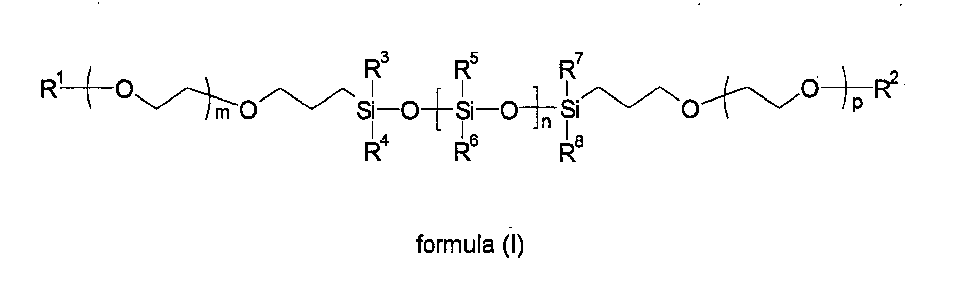 wherein
R¹ and R², independently of one another, are a straight-chain or branched, saturated alkyl group having 6 to 22 carbon atoms or are a straight-chain or branched, mono- or polyunsaturated alkenyl group having 6 to 22 carbon atoms,
R³, R⁴, R⁵, R⁶, R⁷ and R⁸, independently of one another, are a straight-chain or branched, saturated alkyl group having 1 to 30 carbon atoms, a straight-chain or branched, mono- or polyunsaturated alkenyl group having 2 to 30 carbon atoms or a phenyl radical,
m and p are numbers from 4 to 50 and
n is a number from 3 to 90.

2. The linear organopolysiloxane-polyoxyalkylene copolymer as claimed in claim 1, **characterized in that**
R¹ and R², independently of one another, are a straight-chain or branched, saturated alkyl group having 12 to 22 carbon atoms, preferably 16 to 22 carbon atoms, or a straight-chain or branched, mono- or polyunsaturated alkenyl group having 12 to 22 carbon atoms, preferably 16 to 22 carbon atoms, and
R³, R⁴, R⁵, R⁶, R⁷ and R⁸ are each a methyl group.

3. The linear organopolysiloxane-polyoxyalkylene copolymer as claimed in claim 1 or 2, **characterized in that**
m and p are numbers from 5 to 40, preferably 6 to 30 and particularly preferably 7 to 25, and
n is a number from 10 to 60, preferably 20 to 40 and particularly preferably 22 to 30.

4. The linear organopolysiloxane-polyoxyalkylene copolymer as claimed in one or more of claims 1 to 3, **characterized in that**
R¹ and R² are cetearyl,
R³, R⁴, R⁵, R⁶, R⁷ and R⁸ are each a methyl group,
m and p are numbers from 20 to 30, preferably 25, and
n is a number from 20 to 30, preferably 25.

5. The linear organopolysiloxane-polyoxyalkylene copolymer as claimed in claim 1 or 2, **characterized in that**
R¹ and R² are cetearyl,
R³, R⁴, R⁵, R⁶, R⁷ and R⁸ are each a methyl group,
m and p are numbers from 20 to 30, preferably 25, and
n is a number from 70 to 90, preferably 80.

6. A cosmetic or pharmaceutical composition comprising one or more organopolysiloxane-polyoxyalkylene copolymers of the formula (I) as claimed in one or more of claims 1 to 5.

7. The composition as claimed in claim 6, **characterized in that** it is in the form of an emulsion.

8. The composition as claimed in claim 7, **characterized in that** the emulsion is a water-in-oil emulsion, oil-in-water emulsion, microemulsion, nanoemulsion, or multiple emulsion, preferably a water-in-oil emulsion or an oil-in-water emulsion.

9. The composition as claimed in claim 7 or 8, **characterized in that** it comprises the one or more organopolysiloxane-polyoxyalkylene copolymers of the formula (I) in amounts of from 0.01 to 30% by weight, preferably from 0.05 to 10 % by weight and particularly preferably from 0.1 to 5 % by weight, based on the finished composition.

10. The composition as claimed in one or more of claims 6 to 9, **characterized in that** it is in the form of a hair-treatment composition.

11. The composition as claimed in one or more of claims 6 to 9, **characterized in that** it is a deodorant or antiperspirant, preferably in the form of a spray, stick, gel, cream or lotion.

12. The use of one or more linear organopolysiloxane-polyoxyalkylene copolymers of the formula (I) as claimed in one or more of claims 1 to 5, preferably in cosmetic or dermatological compositions, for the cleansing and care of the hair and the skin.

13. The use of one or more linear organopolysiloxane-polyoxyalkylene copolymers of the formula (I) as claimed in one or more of claims 1 to 5 as emulsifier in emulsions, preferably in O/W emulsions or in W/O emulsions.

14. The use of one or more linear organopolysiloxane-polyoxyalkylene copolymers of the formula (I) as claimed in one or more of claims 1 to 5, preferably in cosmetic or dermatological compositions, as conditioner.

15. The use of one or more linear organopolysiloxane-polyoxyalkylene copolymers of the formula (I) as claimed in one or more of claims 1 to 5 as emulsifier and at the same time as conditioner in emulsions, preferably in O/W or W/O emulsions, in particular in those emulsions which are in the form of cosmetic or dermatological compositions.
